# EUROPEAN PATENT APPLICATION

(11) **EP 4 659 725 A1**
(43) Date of publication of application: **10.12.2025**
(21) Application number: 23919886.4
(22) Date of filing: 14.11.2023
(51) Int. Cl.: A61F 13/496

(54) **PANTS-TYPE ABSORBENT ARTICLE**

(30) Priority: 30.01.2023 JP 2023012323
(71) Applicant: DAIO PAPER CORPORATION, Shikokuchuo-shi Ehime 799-0492 (JP)
(72) Inventor: MINAMI, Takeshi, Sakura-shi, Tochigi 329-1411 (JP)
(74) Representative: Bandpay & Greuter
(86) International application number: PCT/JP2023/040842
(87) International publication number: WO 2024/161751

(57) **Abstract**

To easily tear up a pants-type absorbent article along a side seal extending in a longitudinal direction and to stabilize a process of forming the side seal, a pants-type disposable diaper (100) includes a side seal (50) in which a side portion (11) of a ventral portion (10) and a side portion (31) of a dorsal portion (30) are joined, a waist opening (P1), and two leg openings (P2), in which in the side seal (50), at least two columns of a plurality of seal portions (51a) and (52a) arranged in a longitudinal direction (H) are formed in a width direction (W), the seal portions (51a) of a first inner column (51) and the seal portions (52a) of a second inner column (52) are alternately arranged in the longitudinal direction (H), an interval (Dy) between the seal portion (51a) and the seal portion (52a) along the longitudinal direction (H) is set to 0.8 to 1.2 mm, and the seal portions (51 a) and (52a) have a longitudinal dimension (Y) larger than a lateral dimension (X).

## Description

### TECHNICAL FIELD

The present invention relates to a pants-type absorbent article.

### BACKGROUND

In the case of taking off (removing) a pants-type disposable diaper in use, joining regions (side seals) extending in a longitudinal direction at both side portions of a waistline of the diaper are torn up. The side seal is typically formed by arranging a plurality of joining portions (seal portions) in the longitudinal direction.

In the case of tearing up the side seal, the side seal is torn up from a waist opening toward a leg opening, but tearing in the longitudinal direction along the side seal may fail, and the side seal may be torn in a circumferential direction of the waistline, that is, in a lateral direction that is a width direction of the diaper in the middle. When the diaper is torn in the lateral direction, the diaper cannot be smoothly removed.

To address the problem, it has been proposed to devise the shape or arrangement pattern of the seal portions such that the side seal is easily torn up along the longitudinal direction (see, for example, Patent Literatures 1 and 2).

### CITATION LIST

### Patent Literature

Patent Literature 1: JP 2015-096192 A
Patent Literature 2: JP 2022-070524 A

### SUMMARY

### Technical Problem

However, a too narrow interval between the seal portions causes an adhesive such as a hotmelt melted by ultrasonic welding to seep out to the surface side, or forms a region in which a seal pattern that is an assembly of a plurality of seal portions is not present in the lateral direction, making it difficult to control a process of forming the side seal, and making waste of a nonwoven fabric that is a diaper material adhere to equipment used in the process, which lead to a concern that operation of the seal process becomes unstable.

The present invention has been made in view of the above circumstances, and an object of the present invention is to provide a pants-type absorbent article that is easily torn up along a side seal extending in a longitudinal direction when the side seal is torn up from a waist opening toward a leg opening or when the side seal is torn up from the leg opening toward the waist opening, and that can stabilize a process of forming the side seal.

### Solution to Problem

The present invention is a pants-type absorbent article including: a ventral portion, a crotch portion, and a dorsal portion formed to be continuously connected in this order in a predetermined direction in an unfolded state; a joining region in which a side portion of the ventral portion and a side portion of the dorsal portion are joined to overlap each other; a waist opening; and two leg openings, wherein in the joining region, at least two columns of a plurality of joining portions arranged along an extending direction of the joining region are formed in a width direction, of the two columns of the joining portions, a plurality of the joining portions arranged in a first inner column that is a column of the joining portions on an innermost side in the width direction and a plurality of the joining portions arranged in a second inner column that is a column of the joining portions on an outer side in the width direction closest to the first inner column are alternately arranged in the extending direction of the joining region, an interval Dy between the joining portion of the first inner column and the joining portion of the second inner column along the extending direction of the joining region is set to 0.8 to 1.2 mm, and an outline shape of the joining portion of the first inner column and an outline shape of the joining portion of the second inner column have a longitudinal dimension along the extending direction of the joining region larger than a lateral dimension along a direction orthogonal to the extending direction of the joining region.

### Advantageous Effects

The pants-type absorbent article according to the present invention is easily torn up along a side seal extending in a longitudinal direction when the side seal is torn up from a waist opening toward a leg opening or when the side seal is torn up from the leg opening toward the waist opening, and can stabilize a process of forming the side seal.

### BRIEF DESCRIPTION OF DRAWINGS

[Fig. 1] Fig. 1 is a schematic view illustrating a disposable diaper in a state of being worn by a wearer.
[Fig. 2] Fig. 2 is a schematic view illustrating a left joining region (side seal) in a disposable diaper of a first embodiment.
[Fig. 3] Fig. 3 is a schematic view illustrating an example of a vertically-long elliptical shape as an outline shape of a seal portion in a first inner column and a second inner column.
[Fig. 4] Fig. 4 is a schematic view illustrating a state in which rupture progresses in a longitudinal direction H of a side seal 50 by a tearing load.
[Fig. 5] Fig. 5 is a schematic view illustrating a left side seal in a disposable diaper of a second embodiment.
[Fig. 6] Fig. 6 is a schematic view illustrating a left side seal in a disposable diaper of a third embodiment.

### DESCRIPTION OF EMBODIMENTS

Embodiments of an absorbent article according to the present invention will be described below with reference to the drawings.

### [Basic Configuration]

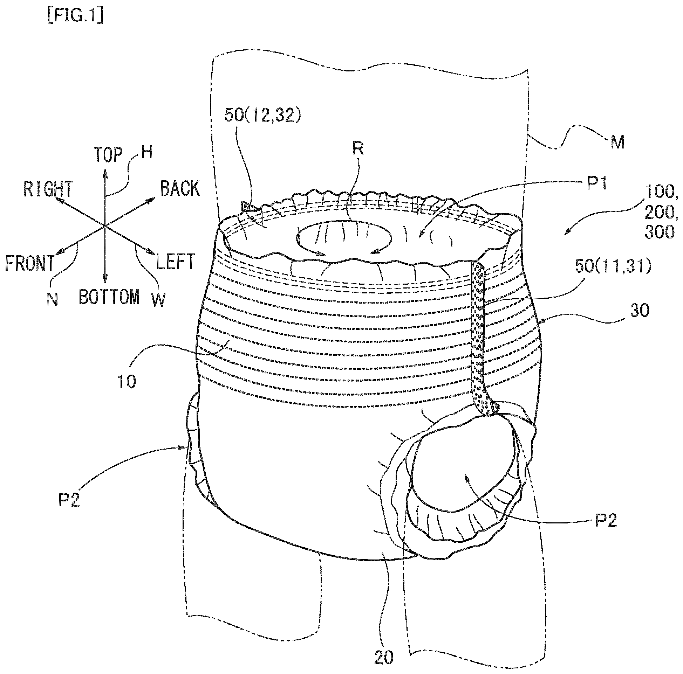

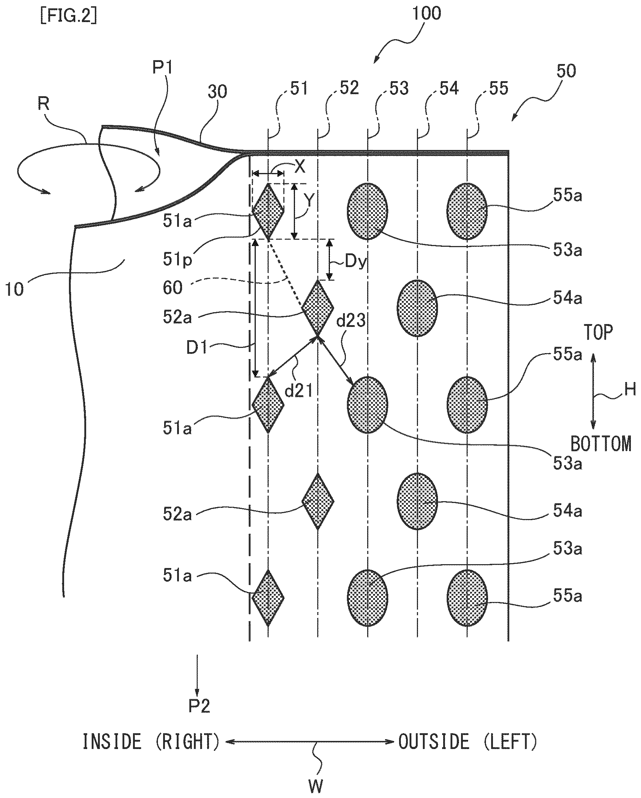

Fig. 1 is a schematic view illustrating a disposable diaper 100, 200, 300 in a state of being worn by a wearer M, and Fig. 2 is a schematic view illustrating a left joining region (side seal) 50 in a disposable diaper 100 of a first embodiment. The disposable diaper 100 is one embodiment of a pants-type absorbent article according to the present invention. In an unfolded state, the disposable diaper 100 is formed by continuously connecting a ventral portion 10, a crotch portion 20, and a dorsal portion 30 in this order in a predetermined direction (length direction).

The disposable diaper 100 includes the joining region (side seal) 50 in which a left side portion 11 in a width direction (lateral direction) W of the ventral portion 10 and a left side portion 31 in the width direction W of the dorsal portion 30 in the state of being unfolded in the length direction are joined, and a joining region (side seal) 50 in which a right side portion 12 in the width direction W of the ventral portion 10 and a right side portion 32 in the width direction W of the dorsal portion 30 are joined. As a result, in the disposable diaper 100, an upper portion in a direction (height direction, longitudinal direction) H in which the side seal 50 extends forms a waist opening P1 in which the ventral portion 10 and the dorsal portion 30 are connected in a circumferential direction R, and a lower portion in the height direction (longitudinal direction) H forms two leg openings P2, so that the entire disposable diaper 100 has a pants shape.

Each of the two leg openings P2 is an opening through which the leg of the wearer M passes in a state where the wearer M wears the disposable diaper 100, and the waist opening P1 is an opening through which the waist of the wearer M passes. In this manner, in a state where the wearer wears the disposable diaper 100, the ventral portion 10 covers the abdomen of the wearer M, the dorsal portion 30 covers the buttocks of the wearer M, and the crotch portion 20 covers the crotch of the wearer M.

Here, the side seal 50 is formed by ultrasonic welding of applying minute vibration while applying pressure in a stacking direction of the ventral portion 10 and the dorsal portion 30 in a stacked state in which an adhesive such as a hotmelt is sandwiched between surfaces of the ventral portion 10 and the dorsal portion 30 overlapping each other.

### (First Embodiment)

In the disposable diaper 100 of the first embodiment, the side seal 50 is formed as described below in the basic configuration described above.

That is, as illustrated in Fig. 2, the side seal 50 on the left side in the width direction W of the disposable diaper 100 includes a column 51 in which a plurality of joining portions (seal portions) 51a are arranged along the extending direction (longitudinal direction H) of the side seal 50, a column 52 in which a plurality of seal portions 52a are arranged along the longitudinal direction H, a column 53 in which a plurality of seal portions 53a are arranged along the longitudinal direction H, a column 54 in which a plurality of seal portions 54a are arranged along the longitudinal direction H, and a column 55 in which a plurality of seal portions 55a are arranged along the longitudinal direction H.

The side seal 50 on the right side in the width direction W of the disposable diaper 100 is formed symmetrically with the side seal 50 on the left side in Fig. 2 in the width direction W. In Fig. 2, the left side in the width direction W is the inner side toward the center of the disposable diaper 100, and the right side in the width direction W is the outer side of the disposable diaper 100.

Here, the column 51 is a column formed on the innermost side in the width direction W in the left side seal 50, and is hereinafter referred to as a first inner column 51. In addition, the column 52 is a column formed on the outer side in the width direction W closest to the first inner column 51, and is hereinafter referred to as a second inner column 52. As will be described later, the first inner column 51 and the second inner column 52 form a guide portion that guides rupture by a tearing load.

In addition, the column 53 is a column formed on the outer side in the width direction W closest to the second inner column 52, and is hereinafter referred to as a third inner column 53. The column 54 is a column formed on the outer side in the width direction W closest to the third inner column 53, and is hereinafter referred to as a fourth inner column 54. The column 55 is a column formed on the outer side in the width direction W closest to the fourth inner column 54, and is hereinafter referred to as a fifth inner column 55.

In the disposable diaper 100, the plurality of seal portions 51a arranged in the first inner column 51 and the plurality of seal portions 52a arranged in the second inner column 52 are alternately arranged in the longitudinal direction H, and an interval Dy, which is a gap between the alternately arranged seal portions 51a and 52a, is set to 0.8 to 1.2 mm.

In addition, in the disposable diaper 100, an outline shape of the seal portions 51a of the first inner column 51 and an outline shape of the seal portions 52a of the second inner column 52 are formed such that a dimension (longitudinal dimension) Y along the longitudinal direction H is larger than a dimension (lateral dimension) X along the width direction W (lateral direction) orthogonal to the longitudinal direction H. Specifically, the outline shapes of the seal portions 51a and 52a are formed in a vertically-long rhombus shape in which the longitudinal dimension Y is larger than the lateral dimension X as illustrated in Fig. 2.

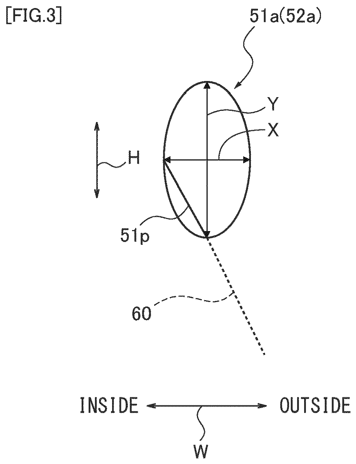

Fig. 3 is a schematic view illustrating an example of a vertically-long elliptical shape as the outline shapes of the seal portions 51a and 52a. In the disposable diaper 100, the outline shapes of the seal portions 51a and 52a only need to be a vertically-long shape (lateral dimension X < longitudinal dimension Y), and are not limited to the vertically-long rhombus shape. Specifically, in the disposable diaper 100, the outline shapes of the seal portions 51a and 52a may be a vertically-long elliptical shape as illustrated in Fig. 3, or may be another vertically-long shape.

In the disposable diaper 100, as illustrated in Fig. 2, the seal portions 53a of the third inner column 53, the seal portions 54a of the fourth inner column 54, and the seal portions 55a of the fifth inner column 55 are formed in a vertically-long elliptical shape. However, the seal portions on the outer side in the width direction W excluding the seal portions 51a of the first inner column 51 and the seal portions 52a of the second inner column 52 do not have to have a vertically-long shape, and may have a shape other than the elliptical shape. In addition, in the disposable diaper 100, the third inner column 53, the fourth inner column 54, and the fifth inner column 55 on the outer side in the width direction W excluding the seal portions 51a of the first inner column 51 and the seal portions 52a of the second inner column 52 may be omitted, or at least one column may be formed.

In each of the above-described seal portions 51a, 52a, 53a, 54a, and 55a, not only outlines of the vertically-long outline shapes are joined, but entire inner ranges surrounded by the outline shapes are also joined.

In addition, in the disposable diaper 100, the longitudinal dimension Y of the seal portion 51a of the first inner column 51 is set to be larger than the interval Dy between the seal portion 51a and the seal portion 52a (Dy < Y).

Furthermore, in the disposable diaper 100, the seal portion 52a of the second inner column is disposed on an extension line 60 of a tear line 51p of the seal portion 51a of the first inner column 51. Here, the tear line 51p is a straight line connecting an end point on the inner side of the lateral dimension X and an end point on the lower side of the longitudinal dimension Y of the seal portion 51a, and in the rhombic seal portion 51a illustrated in Fig. 2, coincides with the outline located on the inner side in the width direction W and on the lower side of the rhombic outline shape.

In a case where the outline shape of the seal portion 51a is a vertically-long elliptical shape illustrated in Fig. 3, the tear line 51p is a straight line connecting an end point on the inner side of the lateral dimension X and an end point on the lower side of the longitudinal dimension Y of the seal portion 51a. In a case where the outline shape of the seal portion 51a is another shape, the tear line 51p is similar to the case of the vertically-long elliptical shape.

Furthermore, in the disposable diaper 100, a distance d21 from the seal portion 52a of the second inner column 52 to the seal portion 51a of the first inner column 51 is set to be shorter than a distance d23 from the seal portion 52a of the second inner column 52 to the seal portion 53a of the third inner column 53 (d21 < d23).

When the wearer M takes off the disposable diaper 100 or a helper takes off the disposable diaper 100 worn by the wearer M, the wearer M or the helper holds the ventral portion 10 near the side seal 50 in the waist opening P1 with one hand, holds the dorsal portion 30 near the side seal 50 in the waist opening P1 with the other hand, and tears up the side seal 50 from the waist opening P1 toward the leg opening P2 by moving both hands away from each other.

This tearing load first acts on the top seal portion 51a of the first inner column 51 in the side seal 50. Then, the tearing load causes the ventral portion 10 or the dorsal portion 30 to rupture along the outline located on the inner side in the width direction of the outline shape of the seal portion 51a. Here, which of the ventral portion 10 and the dorsal portion 30 ruptures depends on the state of sealing of the seal portion 51a, the manner of applying a load at the time of rupture, and the like.

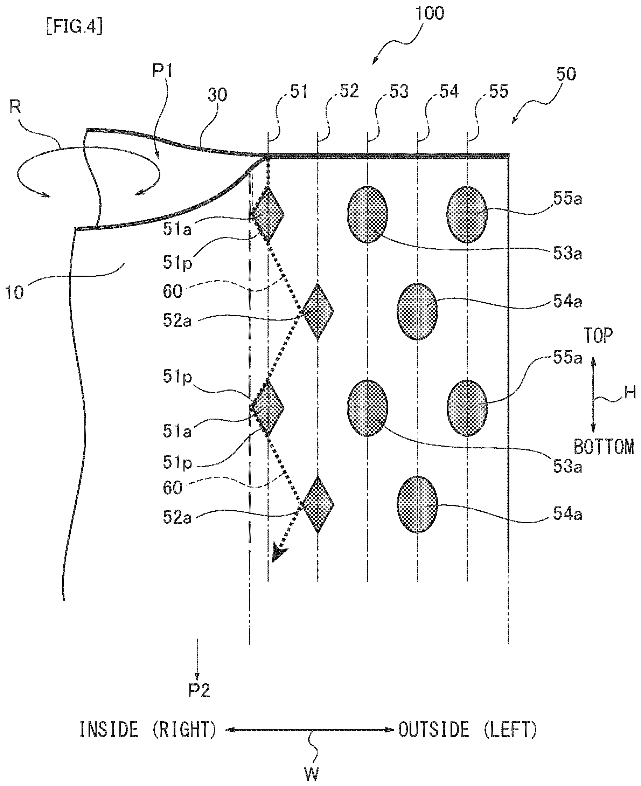

Fig. 4 is a schematic view illustrating a state in which the rupture progresses in the longitudinal direction H of the side seal 50 by the tearing load. As indicated by the short-interval broken line in Fig. 4, the tearing load causes the rupture of the ventral portion 10 or the dorsal portion 30 to progress along the outline on the inner side in the width direction W of the top seal portion 51a of the first inner column 51, and to reach the seal portion 51a of the second inner column 52 formed below the seal portion 51a. Then, the tearing load returns to the inner side in the width direction W at the seal portion 52a and is transmitted to the second seal portion 51a of the first inner column 51 formed below the seal portion 52a.

Subsequently, the tearing load is alternately transmitted to the seal portions 51a of the first inner column 51 and the seal portions 52a of the second inner column 52 from the upper side to the lower side, and as indicated by the short-interval broken line in Fig. 4, the rupture progresses from the upper side to the lower side between the seal portions 51a of the first inner column 51 and the seal portions 52a of the second inner column 52 of the side seal 50, and finally reaches the left leg opening P2, so that the left side seal 50 ruptures in the longitudinal direction H, and the ventral portion 10 and the dorsal portion 30 are separated.

The right side seal 50 also ruptures similarly to the left side seal 50 described above, and the ventral portion 10 and the dorsal portion 30 are separated.

As described above, in the pants-type disposable diaper 100 of the first embodiment, as the column of the plurality of seal portions arranged discontinuously along the longitudinal direction H, at least two columns of the first inner column 51 and the second inner column 52 are formed on both sides in the width direction W, and the plurality of seal portions 51a arranged in the first inner column 51 and the plurality of seal portions 52a arranged in the second inner column 52 are alternately arranged in the longitudinal direction H.

As a result, the disposable diaper 100 can transmit the rupture generated from the periphery of the seal portion 51a of the first inner column 51 to the seal portion 52a of the second inner column 52, making the side seal 50 difficult to rupture in the width direction W and easily torn up along the longitudinal direction H.

In addition, in the disposable diaper 100, since the seal portion 51a of the first inner column 51 and the seal portion 52a of the second inner column 52 to which the tearing load is transmitted are shaped such that the longitudinal dimension Y is larger than the lateral dimension X (lateral dimension X < longitudinal dimension Y), the rupture along the outline of each of the seal portions 51a and 52a is more likely to progress in the longitudinal direction H than in the width direction W, and the tearing load is more likely to be transmitted in the longitudinal direction H, so that the side seal 50 can be easily torn up along the longitudinal direction H.

In addition, in the disposable diaper 100, the interval Dy along the longitudinal direction H between the seal portion 51a of the first inner column 51 and the seal portion 52a of the second inner column 52 alternately arranged in the longitudinal direction H is set to 0.8 to 1.2 mm. As a result, the disposable diaper 100 can prevent the adhesive from leaking from the seal portions 51a and 52a to sonic equipment in a manufacturing process of forming the seal portions 51a and 52a and prevent dirt from adhering to the sonic equipment while making the tearing load easily transmitted in the longitudinal direction H, can prevent the operation of the sonic equipment from becoming unstable due to the adhesion of dirt, and can stabilize the process of forming the side seal 50.

In addition, in the disposable diaper 100, since the longitudinal dimension Y of the seal portion 51a of the first inner column 51 is set to be larger than the interval Dy of the gap between the seal portion 51a of the first inner column 51 and the seal portion 52a of the second inner column 52 (Dy < Y), the tearing load is easily transmitted from the seal portion 51a of the first inner column 51 to the seal portion 52a of the second inner column 52.

In the disposable diaper 100, the longitudinal dimension Y of the seal portion 51a of the first inner column 51 may be set equal to the interval Dy between the seal portion 51a and the seal portion 52a (Dy = Y). In this case as well, the tearing load is easily transmitted from the seal portion 51a of the first inner column 51 to the seal portion 52a of the second inner column 52.

That the longitudinal dimension Y of the seal portion 51a of the first inner column 51 is set to be larger than or equal to the interval Dy of the gap between the seal portion 51a of the first inner column 51 and the seal portion 52a of the second inner column 52 (Dy ≤ Y) is a preferable aspect, but is not an essential element in the pants-type absorbent article according to the present invention.

In addition, in the disposable diaper 100, since the seal portion 52a of the second inner column 52 is disposed on the extension line 60 of the tear line 51p of the seal portion 51a of the first inner column 51, the tearing load is easily transmitted from the seal portion 51a of the first inner column 51 to the seal portion 52a of the second inner column 52.

That the seal portion 52a of the second inner column 52 is disposed on the extension line 60 of the tear line 51p of the seal portion 51a of the first inner column 51 is a preferable aspect, but is not an essential element in the pants-type absorbent article according to the present invention.

In addition, in the disposable diaper 100, the distance d21 from the seal portion 52a of the second inner column 52 to the seal portion 51a of the first inner column 51 is shorter than the distance d23 from the seal portion 52a of the second inner column 52 to the seal portion 53a of the third inner column 53. Therefore, the rupture that has progressed from the seal portion 51a of the first inner column 51 to the seal portion 52a of the second inner column 52 can more easily progress to the seal portion 51a of the first inner column 51 from the seal portion 52a of the second inner column 52 than to the seal portion 53a of the third inner column 53.

That the distance d21 from the seal portion 52a of the second inner column 52 to the seal portion 51a of the first inner column 51 is shorter than the distance d23 from the seal portion 52a of the second inner column 52 to the seal portion 53a of the third inner column 53 is a preferable aspect, but is not an essential element in the pants-type absorbent article according to the present invention.

### (Second Embodiment)

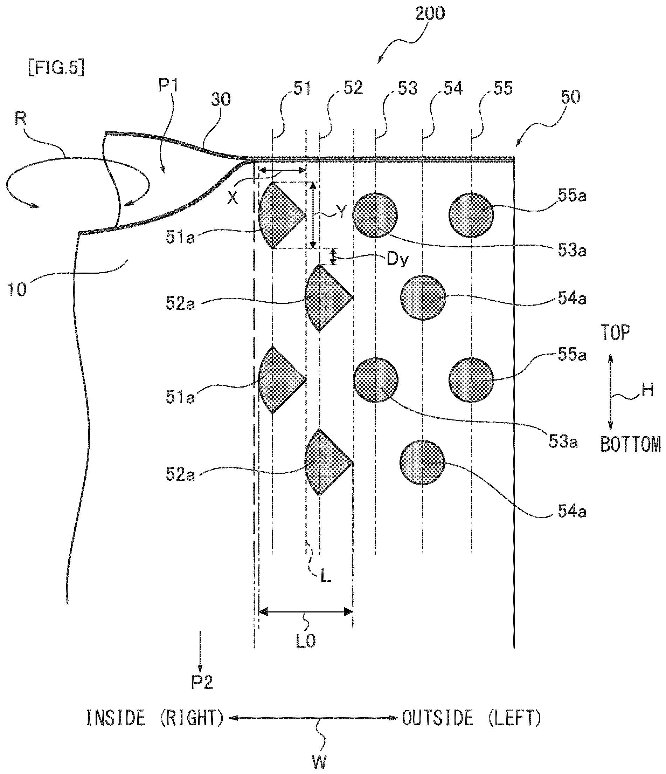

Fig. 5 is a schematic view illustrating a left side seal 50 in a pants-type disposable diaper 200 of a second embodiment. The pants-type disposable diaper 200 of the second embodiment has the same basic configuration as that of the disposable diaper 100 of the first embodiment described above.

Specifically, as illustrated in Fig. 5, in the side seal 50 on the left side in the width direction W of the disposable diaper 200, a first inner column 51, a second inner column 52, a third inner column 53, a fourth inner column 54, and a fifth inner column 55 are formed sequentially from the inner side in the width direction W.

In the disposable diaper 200, a plurality of seal portions 51a are arranged along the longitudinal direction H in the first inner column 51, a plurality of seal portions 52a are arranged along the longitudinal direction H in the second inner column 52, a plurality of seal portions 53a are arranged along the longitudinal direction H in the third inner column 53, a plurality of seal portions 54a are arranged along the longitudinal direction H in the fourth inner column 54, and a plurality of seal portions 55a are arranged along the longitudinal direction H in the fifth inner column 55.

In the disposable diaper 200, the plurality of seal portions 51a arranged in the first inner column 51 and the plurality of seal portions 52a arranged in the second inner column 52 are alternately arranged in the longitudinal direction H, and an interval Dy, which is a gap between the alternately arranged seal portions 51a and 52a, is set to 0.8 to 1.2 mm.

In addition, in the disposable diaper 200, an outline shape of the seal portions 51a of the first inner column 51 and an outline shape of the seal portions 52a of the second inner column 52 are formed such that a dimension (longitudinal dimension) Y along the longitudinal direction H is larger than a dimension (lateral dimension) X along the width direction W (lateral direction) orthogonal to the longitudinal direction. Specifically, the outline shapes of the seal portions 51a and 52a are formed in a lateral fan shape.

Specifically, in the lateral fan shape of the seal portions 51a and 52a, a line directed to the inner side in the width direction W is an arc, and a line directed to the outer side in the width direction is formed by a straight line inclined downward in the longitudinal direction H from an upper end of the arc toward the outer side in the width direction W and a straight line inclined upward in the longitudinal direction H from a lower end of the arc toward the outer side in the width direction W intersecting each other at an angle of 90 degrees, for example.

In the disposable diaper 200, the seal portions 53a of the third inner column 53, the seal portions 54a of the fourth inner column 54, and the seal portions 55a of the fifth inner column 55 are formed in a circular shape, but the third inner column 53, the fourth inner column 54, and the fifth inner column 55 may be omitted.

In addition, in the disposable diaper 200, dimensions between the first inner column 51 and the second inner column 52 are set such that at least either the seal portion 51a or 52a is formed in any portion of the longitudinal direction H over an entire range L0 in the width direction W from the seal portion 51a of the first inner column 51 to the seal portion 52a of the second inner column 52.

That is, in Fig. 5, in the disposable diaper 200, when a straight line L extending along the longitudinal direction H is virtually moved in the entire range L0 in the width direction W from the seal portion 51a of the first inner column 51 to the seal portion 52a of the second inner column 52, the seal portions 51a and 52a are disposed such that the straight line L passes through at least one of the seal portion 51a or the seal portion 52a over the entire range L0.

Similarly to the disposable diaper 100 of the first embodiment, the pants-type disposable diaper 200 of the second embodiment configured as described above can transmit the rupture generated from the periphery of the seal portion 51a of the first inner column 51 to the seal portion 52a of the second inner column 52, making the side seal 50 difficult to rupture in the width direction W and easily torn up along the longitudinal direction H.

In addition, in the disposable diaper 200, since the seal portion 51a of the first inner column 51 and the seal portion 52a of the second inner column 52 to which the tearing load is transmitted are shaped such that the longitudinal dimension Y is larger than the lateral dimension X (lateral dimension X < longitudinal dimension Y), the rupture along the outline of each of the seal portions 51a and 52a is more likely to progress in the longitudinal direction H than in the width direction W, and the tearing load is more likely to be transmitted in the longitudinal direction H, so that the side seal 50 can be easily torn up along the longitudinal direction H.

In addition, in the disposable diaper 200, the interval Dy along the longitudinal direction H between the seal portion 51a of the first inner column 51 and the seal portion 52a of the second inner column 52 alternately arranged in the longitudinal direction H is set to 0.8 to 1.2 mm. As a result, the disposable diaper 200 can prevent the adhesive from leaking from the seal portions 51a and 52a to sonic equipment in a manufacturing process of forming the seal portions 51a and 52a and prevent dirt from adhering to the sonic equipment while making the tearing load easily transmitted in the longitudinal direction H, can prevent the operation of the sonic equipment from becoming unstable due to the adhesion of dirt, and can stabilize the process of forming the side seal 50.

In addition, in the disposable diaper 200, dimensions between the first inner column 51 and the second inner column 52 are set such that at least either the seal portion 51a or 52a is formed in any portion of the longitudinal direction H over the entire range L0 in the width direction W from the seal portion 51a of the first inner column 51 to the seal portion 52a of the second inner column 52.

Here, suppose that a side seal of a disposable diaper having a portion with no seal portion in the entire longitudinal direction H at any position of the entire range L0 in the width direction W from the seal portion 51a of the first inner column 51 to the seal portion 52a of the second inner column 52 is formed. In this case, when the disposable diaper relatively moving in the width direction W is pressurized by a horn of the sonic equipment for ultrasonic welding extending along the longitudinal direction H, it is necessary to finely perform control such as weakening a pressurizing force in the portion with no seal portion in the entire longitudinal direction H, which may result in unstable operation of the sonic equipment.

On the other hand, since the disposable diaper 200 of the second embodiment does not have a portion with no seal portion in the entire longitudinal direction H at all positions of the entire range L0 in the width direction W, the operation of the sonic equipment is not made unstable as described above, and the sonic equipment can be stably operated.

That there is no portion with no seal portion in the entire longitudinal direction H at all positions of the entire range L0 in the width direction W is a preferable aspect, but is not an essential element in the pants-type absorbent article according to the present invention.

In addition, in the disposable diaper 200, since the seal portion 51a is formed in the lateral fan shape in which the line directed to the inner side in the width direction W is the arc, the tearing load is easily transmitted from the seal portion 51a of the first inner column 51 to the seal portion 52a of the second inner column 52.

In the disposable diaper 200, a crescent shape protruding toward the inner side in the width direction W can be adopted as the seal portion 51a instead of the lateral fan shape in which the line directed to the inner side in the width direction W is the arc.

### (Third Embodiment)

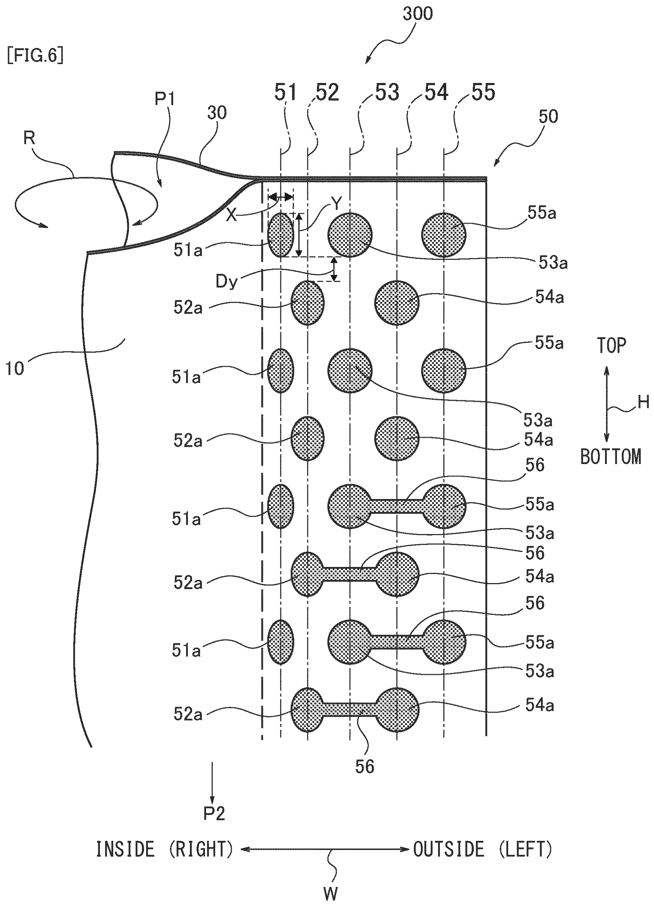

Fig. 6 is a schematic view illustrating a left side seal 50 in a pants-type disposable diaper 300 of a third embodiment. The pants-type disposable diaper 300 of the third embodiment has the same basic configuration as that of the disposable diaper 100 of the first embodiment described above.

Specifically, as illustrated in Fig. 6, in the side seal 50 on the left side in the width direction W of the disposable diaper 300, a first inner column 51, a second inner column 52, a third inner column 53, a fourth inner column 54, and a fifth inner column 55 are formed sequentially from the inner side in the width direction W.

In the disposable diaper 300, a plurality of seal portions 51a are arranged along the longitudinal direction H in the first inner column 51, a plurality of seal portions 52a are arranged along the longitudinal direction H in the second inner column 52, a plurality of seal portions 53a are arranged along the longitudinal direction H in the third inner column 53, a plurality of seal portions 54a are arranged along the longitudinal direction H in the fourth inner column 54, and a plurality of seal portions 55a are arranged along the longitudinal direction H in the fifth inner column 55.

In the disposable diaper 300, the plurality of seal portions 51a arranged in the first inner column 51 and the plurality of seal portions 52a arranged in the second inner column 52 are alternately arranged in the longitudinal direction H, and an interval Dy, which is a gap between the alternately arranged seal portions 51a and 52a, is set to 0.8 to 1.2 mm.

In addition, in the disposable diaper 300, an outline shape of the seal portions 51a of the first inner column 51 and an outline shape of the seal portions 52a of the second inner column 52 are formed such that a dimension (longitudinal dimension) Y along the longitudinal direction H is larger than a dimension (lateral dimension) X along the width direction W (lateral direction) orthogonal to the longitudinal direction. Specifically, the outline shapes of the seal portions 51a and 52a are formed in a vertically-long elliptical shape.

In addition, in the disposable diaper 300, the seal portions 53a of the third inner column 53, the seal portions 54a of the fourth inner column 54, and the seal portions 55a of the fifth inner column 55 are formed in a circular shape.

In the disposable diaper 300, as illustrated in Fig. 6, some of the plurality of seal portions 52a of the second inner column 52 are connected to and integrally formed with the seal portions 54a of the fourth inner column 54 formed at the same position in the longitudinal direction H by laterally-long belt-shaped seal portions 56. Similarly, in the disposable diaper 300, some of the plurality of seal portions 53a of the third inner column 53 are connected to and integrally formed with the seal portions 55a of the fifth inner column 55 formed at the same position in the longitudinal direction H by the laterally-long belt-shaped seal portions 56.

In the disposable diaper 300, some of the plurality of seal portions 51a of the first inner column 51 may be connected to and integrally formed with the seal portions 53a of the third inner column 53 formed at the same position in the longitudinal direction H by the laterally-long belt-shaped seal portions 56.

Similarly to the disposable diaper 100 of the first embodiment, the pants-type disposable diaper 300 of the third embodiment configured as described above can transmit the rupture generated from the periphery of the seal portion 51a of the first inner column 51 to the seal portion 52a of the second inner column 52, making the side seal 50 difficult to rupture in the width direction W and easily torn up along the longitudinal direction H.

In addition, in the disposable diaper 300, since the seal portion 51a of the first inner column 51 and the seal portion 52a of the second inner column 52 to which the tearing load is transmitted are shaped such that the longitudinal dimension Y is larger than the lateral dimension X (lateral dimension X < longitudinal dimension Y), the rupture along the outline of each of the seal portions 51a and 52a is more likely to progress in the longitudinal direction H than in the width direction W, and the tearing load is more likely to be transmitted in the longitudinal direction H, so that the side seal 50 can be easily torn up along the longitudinal direction H.

In addition, in the disposable diaper 300, the interval Dy along the longitudinal direction H between the seal portion 51a of the first inner column 51 and the seal portion 52a of the second inner column 52 alternately arranged in the longitudinal direction H is set to 0.8 to 1.2 mm. As a result, the disposable diaper 300 can prevent the adhesive from leaking from the seal portions 51a and 52a to sonic equipment in a manufacturing process of forming the seal portions 51a and 52a and prevent dirt from adhering to the sonic equipment while making the tearing load easily transmitted in the longitudinal direction H, can prevent the operation of the sonic equipment from becoming unstable due to the adhesion of dirt, and can stabilize the process of forming the side seal 50.

Furthermore, in the disposable diaper 300, some of the plurality of seal portions 52a of the second inner column 52 are integrally formed with the seal portions 54a of the fourth inner column 54, and some of the plurality of seal portions 53a of the third inner column 53 are integrally formed with the seal portions 55a of the fifth inner column 55.

Therefore, since the disposable diaper 300 does not have a portion with no seal portion in the entire longitudinal direction H at any position of an entire range in the width direction W from the seal portion 52a of the second inner column 52 to the seal portion 54a of the fourth inner column 54, nor a portion with no seal portion in the entire longitudinal direction H at any position of an entire range in the width direction W from the seal portion 53a of the third inner column 53 to the seal portion 55a of the fifth inner column 55, the operation of the sonic equipment is not made unstable, and the sonic equipment can be stably operated due to a reason similar to that in the disposable diaper 200 of the second embodiment.

In the disposable diaper 300, in a case where some of the seal portions 51a of the first inner column 51 are integrally formed with the seal portions 53a of the third inner column 53, there is no portion with no seal portion in the entire longitudinal direction H at any position of an entire range in the width direction W from the seal portion 51a of the first inner column 51 to the seal portion 53a of the third inner column 53, so that the sonic equipment can be stably operated in the entire region of the side seal 50.

In addition, in the disposable diaper 300, the seal portions at the same position in the longitudinal direction H are connected to each other to form a large seal portion, so that the joining strength of the portion can be increased.

In the disposable diaper 300, the selection of the seal portion 52a to be integrally formed with the seal portion 54a of the fourth inner column 54, from the plurality of seal portions 52a of the second inner column 52, may be set according to the number of overlapped constituent members of the ventral portion 10 and the dorsal portion 30. That is, in the disposable diaper 300, a joining portion having a large number of overlapped sheets to be joined by the seal portion has a higher joining strength than a joining portion having a small number of overlapped sheets.

To tear up and rupture the portion having a high joining strength in the disposable diaper 300, it is necessary to apply a large tearing load to the portion having a high joining strength. Therefore, in the disposable diaper 300, it is preferable to inhibit the joining strength from increasing by reducing the joining area of the joining portion having a large number of overlapped sheets as compared with the joining portion having a small number of overlapped sheets, to facilitate tearing.

Therefore, in the disposable diaper 300, it is preferable that the seal portions are not connected in the portion having a large number of overlapped sheets to be joined by the seal portion in order to reduce the joining area as compared with the portion having a small number of overlapped sheets, and on the other hand, the portion having a small number of overlapped sheets to be joined by the seal portion is selected as a portion connecting the seal portions because the portion does not cause the above-described problem even if the joining area is increased as compared with the portion having a large number of overlapped sheets.

Connecting the seal portions is a preferable aspect, but is not an essential element in the pants-type absorbent article according to the present invention.

The disposable diaper 100, 200, 300 of each of the first, second, and third embodiments described above has been described as being easily torn up along the side seal 50 extending in the longitudinal direction H when the side seal 50 is torn up from the waist opening P1 toward the leg opening P2, and as stabilizing the process of forming the side seal 50. When the side seal 50 is torn up from the leg opening P2 toward the waist opening P1, the disposable diaper 100, 200, 300 is also easily torn up along the side seal 50 extending in the longitudinal direction H, and can also stabilize the process of forming the side seal 50.

### CROSS-REFERENCE TO RELATED APPLICATIONS

This application claims priority based on Japanese Patent Application No. 2023-012323 filed with the Japan Patent Office on January 30, 2023, the entire disclosure of which is entirely incorporated herein by reference.

## Claims

1. A pants-type absorbent article comprising: a ventral portion, a crotch portion, and a dorsal portion formed to be continuously connected in this order in a predetermined direction in an unfolded state; a joining region in which a side portion of the ventral portion and a side portion of the dorsal portion are joined to overlap each other; a waist opening; and two leg openings, wherein
in the joining region, at least two columns of a plurality of joining portions arranged along an extending direction of the joining region are formed in a width direction,
of the two columns of the joining portions, a plurality of the joining portions arranged in a first inner column that is a column of the joining portions on an innermost side in the width direction and a plurality of the joining portions arranged in a second inner column that is a column of the joining portions on an outer side in the width direction closest to the first inner column are alternately arranged in the extending direction of the joining region,
an interval between the joining portion of the first inner column and the joining portion of the second inner column along the extending direction of the joining region is set to 0.8 to 1.2 mm, and
an outline shape of the joining portion of the first inner column and an outline shape of the joining portion of the second inner column have a longitudinal dimension along the extending direction of the joining region larger than a lateral dimension along a direction orthogonal to the extending direction of the joining region.

2. The pants-type absorbent article according to claim 1, wherein the longitudinal dimension of the joining portion of the first inner column is larger than or equal to the interval.

3. The pants-type absorbent article according to claim 1, wherein the joining portion of the second inner column is disposed on an extension line of a tear line of the joining portion of the first inner column.

4. The pants-type absorbent article according to claim 1, wherein a distance from the joining portion of the second inner column to the joining portion of the first inner column is shorter than a distance from the joining portion of the second inner column to a joining portion of a third inner column that is a column of joining portions on an outer side in the width direction of the second inner column.

5. The pants-type absorbent article according to claim 1, wherein at least one of the joining portion of the first inner column or the joining portion of the second inner column and the joining portion at a same position in the extending direction of the joining region in another column of the joining portions different from the first inner column or in another column of the joining portions different from the second inner column are connected and integrally formed by a belt-shaped joining portion.

6. The pants-type absorbent article according to any one of claims 1 to 5, wherein at least one of the joining portions is formed in the extending direction of the joining region over an entire range in the width direction from the joining portion of the first inner column to the joining portion of the second inner column.
